# EUROPEAN PATENT APPLICATION

(11) **EP 2 204 107 A1**
(43) Date of publication of application: **07.07.2010**
(21) Application number: 08842711.7
(22) Date of filing: 21.10.2008
(51) Int. Cl.: A47C 27/00, A47C 21/04, A47G 9/02

(54) **BEDDING IN WHICH TEMPERATURE-CONTROLLED AIR CIRCULATES**

(30) Priority: 26.10.2007 JP 2007278523
(71) Applicant: Toshiba Consumer Electronics Holdings Corporation, Tokyo 101-0021 (JP); Toshiba Home Appliances Corporation, Tokyo 101-0021 (JP)
(72) Inventor: SAKAMOTO, Noriaki, Tokyo 105-8001 (JP); OZAKI, Tatsuya, Tokyo 105-8001 (JP); OCHIAI, Koichiro, Tokyo 105-8001 (JP); ADACHI, Kosaku, Tokyo 105-8001 (JP); EMURA, Yuji, Osaka 532-0012 (JP); ISHIKAWA, Hiroya, Osaka 532-0012 (JP)
(74) Representative: Maury, Richard Philip
(86) International application number: PCT/JP2008/069057
(87) International publication number: WO 2009/054383

(57) **Abstract**

Provided is temperature-controlled air circulation type bedding which introduces the blowoff air generated by a temperature control unit into an air flow passage provided around a bedding body so as to cool or warm the body of a person in the bedding, controls the blowoff air temperature to form a comfortable sleeping environment irrespective of an external atmosphere temperature, suppresses discharge of carbon dioxide gas, etc. with a compact configuration, and has low power consumption. The temperature-controlled air circulation type bedding includes a temperature control unit 2 which controls the blowoff air temperature by a cooling or heating action, and a bedding body 3 which provides an air flow passage 27 which allows the blowoff air from the temperature control unit to be introduced and circulated therethrough, and cools or warms the inside thereof. The temperature of air which circulates through the bedding is detected so that the temperature of the blowoff air is controlled by the temperature control unit.

## Description

### Technical Field

The present invention relates to temperature-controlled air circulation type bedding around which an air flow passage which allows the blowoff air from a temperature control unit to be introduced and circulated therethrough is provided so as to perform cooling or warming of the inside.

### Background Art

Conventionally, in order to obtain comfortable sleeping, people have performed a control by using bedding which is thick and has high heat insulation performance or by using bedding which is thin and has high moisture absorbency according to seasons or surrounding temperature or humidity. Moreover, on high-temperature and high-humidity sultry nights in summer, an air-conditioner has been operated to control the atmospheric air of the room itself to secure comfortable sleeping. Additionally, when a person has a nap in an automobile, in a truck or a passenger car, comfortable sleeping has been secured by cooling by a car air-conditioner with an engine being brought into an idling state.

In the above conventional methods, year-round adjustment of the bedding is complicated, power consumption becomes large in the air conditioning of the whole room by the operation of an air-conditioner, and an idling operation is needed for cooling in an automobile. These also have a great influence on global warming accompanying not only power consumption but discharge of much carbon dioxide gas. For this reason, although the idling stop of the automobile has recently been greatly requested as an available measure for the reduction of carbon dioxide gas, practically, visible accomplishments do not appear, but a great environmental problem is caused.

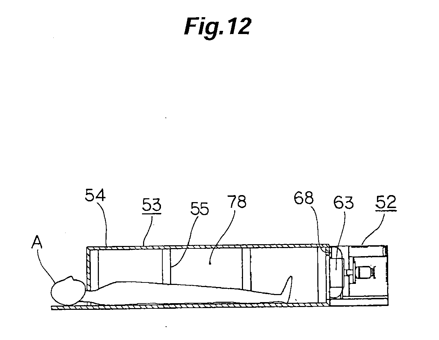

Meanwhile, as shown in Fig. 12, Patent Citation 1 discloses a configuration in which the air from an air conditioner (52) which has a heat exchanger (63) and a blower(68) is circulated into a sleeping bag (53) in which a space (78) where a human being (A) lies is formed so that the inside of the space is cooled, and thereby, a refrigerating machine itself is operated with small electric power so that waste of an energy resource and environment pollution are suppressed.

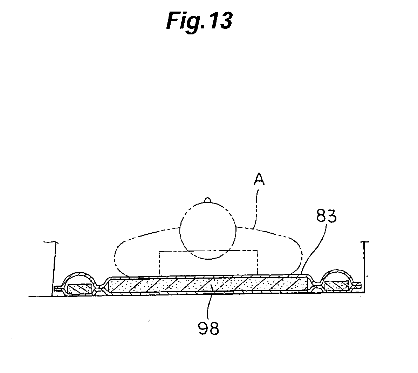

Additionally, Patent Citation 2 shown in Fig. 13 discloses the structure of an air circulation type mat (98) in which a heat exchanger is provided at one longitudinal end of a mat (83) which extends flatly and horizontally so as to supply heat-exchange air, and the supplied air flows through a breathable mat so that a comfortable feeling for a human body (A) is obtained.
Patent Citation 1: JP-A-2007-098044
Patent Citation 2: JP-A-2007-105084

### Disclosure of the Invention

### Problems that the Invention is to Solve

However, the space (78) where a human being lies in the sleeping bag (53) described in Patent Citation 1 is formed by a frame (55) provided inside an insulated sheet (54). Since energy saving has been achieved by forming the space where the human being enters as small as possible to a required minimum, this sleep is greatly different from a sleep in Japanese-style bedding, and is different in terms of feeling or deep sleep effect. Additionally, since the configuration described in Patent Citation 2 is a mat, i.e., matting, this is also different from sleeping in Japanese-style bedding similarly to the above. Additionally, the configurations shown in these Patent Citations 1 and 2 merely deliver and circulate the air which has exchanged heat to the blower, and do not have a temperature control function, so that control to an environmental atmosphere according to the liking of an individual could not be made, and it was difficult to secure comfort.

The invention was made in consideration of the above circumstances, and the object of the invention is to provide temperature-controlled air circulation type bedding which introduces the blowoff air generated by a temperature control unit into an air flow passage provided around a bedding body so as to cool or warm the body of a person in the bedding, controls the blowoff air temperature to form a comfortable sleeping environment irrespective of external atmosphere temperature, suppresses discharge of carbon dioxide gas, etc. with a compact configuration, and has low power consumption.

### Means for Solving the Problems

In order to solve the above problems, the temperature-controlled air circulation type bedding of the invention includes a temperature control unit which controls the blowoff air temperature by a cooling or heating action, and a bedding body which is provided with a circumferential air flow passage which allows the blowoff air from the temperature control unit to be introduced and circulated therethrough, and cools or warms the inside, thereof. The temperature of the air which circulates through the bedding is detected so that the temperature of the blowoff air is controlled by the temperature control unit.

### Advantage of the Invention

According to the invention, the blowoff air temperature to the air flow passage to the periphery of the bedding is controlled to cool or warm the body of a person in the bedding from the periphery, so that a comfortable sleeping environment can be formed irrespective of an external atmosphere temperature, and bedding which is compact and has low electric power consumption can be obtained.

### Best Mode for Carrying out the Invention

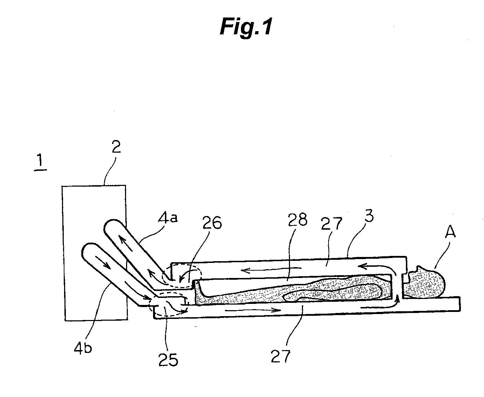

One embodiment of the invention will now be described with reference to the drawings. Temperature-controlled air circulation type bedding (1) shown in the schematic perspective view of Fig. 1 includes a temperature control unit (2), a bedding body (3), a coupling duct (4) which couples the bedding body (3) and the temperature control unit (2).

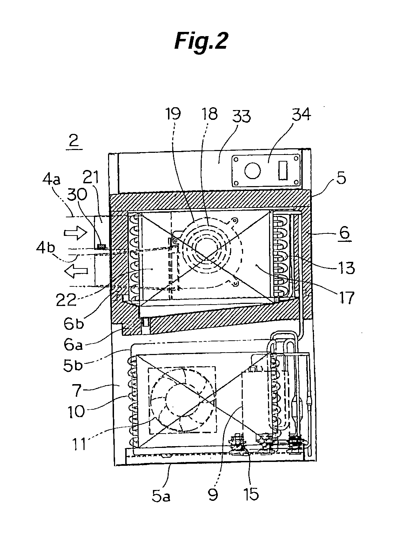

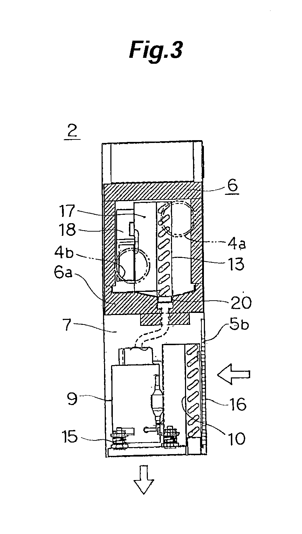

As shown in Fig. 2 which is a sectional view from the front, and Fig. 3 which is a side sectional view of Fig. 2, the temperature control unit (2) forms a box-like outer shell by an outer plate (5) made from a thin steel sheet, and is partitioned into upper and lower spaces substantially at a central portion in a height direction by a heat-insulating partition wall (6a). A bottom portion of the unit, for example, is bolt-fixed to an upper portion of the bottom face of a vehicle body in a rear cabin of a driver's seat of a truck via a mount.

A compressor (9) which compresses and discharges refrigerant which forms part of a refrigeration cycle (8), a condenser (10) which receives, radiates, and condenses the discharged high-temperature and high-pressure refrigerant, and a radiation fan (11) which cools high-temperature components, such as the compressor (9) and the condenser (10), are located on a bottom plate (5a), which is a rigid body which forms a bottom portion of the outer plate (5), thereby forming a machine chamber (7).

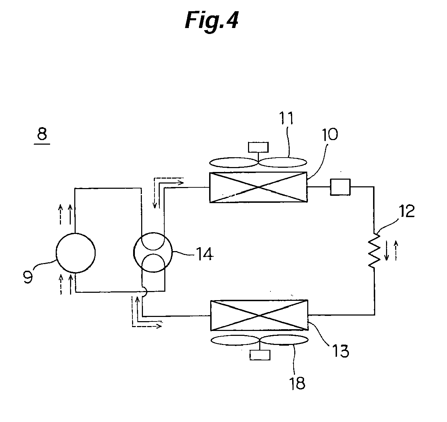

As shown in Fig. 4, the refrigeration cycle (8) is of a vapor compression type in which the compressor (9), the condenser (10), a capillary (12) that is a decompressor, and an evaporator (13) are annularly connected, and the evaporator (13) is made to function as a heat exchanger capable of switching cooling and heating by providing a four-way valve (14) so as to connect together the upstream and downstream of the compressor (9), thereby switching refrigerant flow passages, and in which refrigerant is circulated by the driving of the compressor (9) and is evaporated in the evaporator (13) to generate cool air, or the refrigerant flow passages are switched by the four-way valve (14), and condensation is caused in the evaporator (13) to make the evaporator (13) function as a higher temperature heat exchanger to generate warm air.

The compressor (9) which is a heavy load in the machine chamber (7) is biased toward one side in a width direction on the bottom plate (5a), and is fixed via vibration-absorbing cushion bodies (15). The rectangular parallelepiped-shaped condenser (10) in which a meandered refrigerant pipe is fitted into a number of fins, and the depth dimension which is made small is erected along a suction opening (5b) in which the front face of the condenser having a large area is formed on the front face of the outer plate (5), and a filter (16) is provided between the suction opening (5b) and the condenser (10) so as to shield from dust, etc.

The radiation fan (11) including an axial flow fan is arranged on the back face of the condenser (10). When a cooling operation is performed, the compressor (9) and the radiation fan (11) are synchronously driven, and ambient air is sucked into the inside from the suction opening (5b) to cool the condenser (10) which has a high temperature, and the air which has performed heat exchange is radiated to the outside of the unit from an exhaust port formed in the bottom face of the outer plate (5). At this time, since the compressor (9) is positioned at a side portion in the machine chamber (7), and the compressor hardly receives a cooling action directly by the radiation fan (11), a drop in evaporation temperature caused by supercooling is prevented. Further, the depth dimension of the machine chamber (7) is shortened by arranging the compressor (9) and the radiation fan (11) so as not to overlap each other in the depth direction by a biased distance.

An upper space from the heat-insulating partition wall (6a) is used as a temperature control chamber (17) which generates cooled air or warmed air as a heat-insulating space in which a whole peripheral wall including a top face is formed by the heat-insulating wall (6), such as styrene foam. The evaporator (13) which is a portion of the refrigeration cycle (8) and which receives the refrigerant from the condenser (10) to evaporate the refrigerant during the cooling operation, thereby lowering the temperature of the refrigerant to generate cool air is arranged in an erected state substantially in the central portion of the space.

Similarly to the condenser (10), the evaporator (13) also assumes a rectangular parallelepiped shaped in which the depth dimension is made small from a meandered refrigerant pipe and a number of fins fitted into the refrigerant pipe. At the back of the evaporator, the blower fan (18) including a sirocco fan which has a diameter narrower than the width dimension of the evaporator (13), and a casing (19) are erected abreast of the evaporator (13) at a flat surface portion of the heat-insulating partition wall (6a).

The top face of the heat-insulating partition wall (6a) on which the evaporator (13) is placed forms a gutter portion (20) which is inclined downward toward one side, and receives melting water of the frost adhering to the evaporator (13) the temperature of which has become a low temperature, and the water collected in the gutter portion (20) is drained to the outside via the inside of the lower machine chamber (7).

A return air trunk (21), which is connected to a return duct (4a) of the coupling duct (4) with a bedding body (3) that is an object to be temperature-controlled which is arranged on the bottom face of the vehicle body so as to face the heat-insulating side wall (6b) and which sucks cool air into the temperature control chamber (17), is opened to an upper wall portion which is positioned slightly ahead of the erected position of the evaporator (13) in the heat-insulating side wall (6b) which forms one side wall of the temperature control chamber (17), and a blowoff air trunk (22) is opened from the casing (19) of the blower fan (18) so that the cool air is introduced into an intake port (25) as cool air for the bedding body (3) via a blowoff duct (4b) which leads to the blowoff air trunk (22). In addition, the tip of the return duct (4a) is connected to a return port (26) of the bedding body (3).

At this time, the return air trunk (21) and the blowoff air trunk (22) are opened while being shifted to positions where their axial centers in the vertical height direction and the depth direction of the heat-insulating side wall (6b) of the temperature control chamber (17) are made not to coincide with each other, the two openings (21) and (22) are disposed apart from each other, and the depth dimension as the temperature control unit (2) is made small.

The bedding body (3) has a so-called sleeping bag-like appearance, has an outer surface formed from a heat-insulating body and an inner surface formed from a flexible raw material which allows the passage of moisture, such as sweat, but does not allow the passage of air, and is adapted such that an air flow passage (27) which introduces and circulates the blowoff air from the temperature control unit (2) is provided between the outer surface and the inner surface to cool or warm the inside by a cooling or a warming operation.

Accordingly, if the heat insulation performance of the outer surface of the bedding body (3) is improved, heat leakage decreases. As a result, internal temperature fluctuations can be made small, and cooling or warming effects can be more efficiently obtained. Additionally, a coldness storage material, etc. may be disposed at a suitable portion of the bedding body (3) so as to suppress the range of temperature fluctuations, and the shape of the bedding body is also not limited to the sleeping bag shape, but may be only a lying mat or a sitting mat, or may be an integral configuration of the lying mat and the sitting mat, so long as the bedding body has an air flow passage through at least a portion of which air circulates.

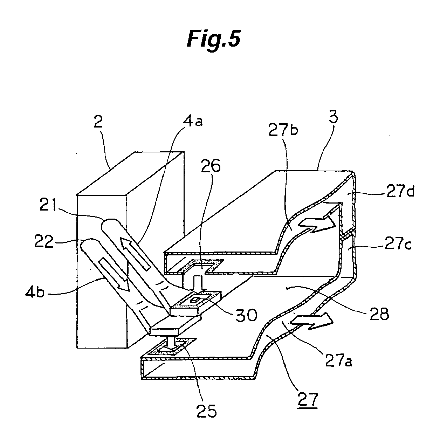

The air flow passage (27) shown in Fig. 5 in which the chief parts are enlarged is provided with a bottom face (27a) and a top face (27b), and right and left side wall portions (27c) so as to form the peripheral wall surface of a space (28) that a user (A) enters, and is formed so as to perform circulation such that air, for example the cool air from the blowoff duct (4b) of the coupling duct, is introduced into the bottom face (27a), and the plurality of flow passages (27c) below both sides walls, is made to flow down in one direction to a longitudinal tip portion of the bedding body (3), then moves and flows forward toward a top rear face (27b), and upper flow passages (27d) of both the side walls, and returns to the temperature control chamber (17) via the return duct (4a).

The blowoff duct (4b) and return duct (4a) of the coupling duct (4) are formed from a bellows-like tubular body so that the length dimension thereof can be adjusted, extends so as to incline toward the lower front side from one side of the temperature control chamber (17) of the temperature control unit (2), and is coupled with the intake port (25) and return port (26) which are provided at a longitudinal one end of the bedding body (3) installed at the bottom face of the vehicle body.

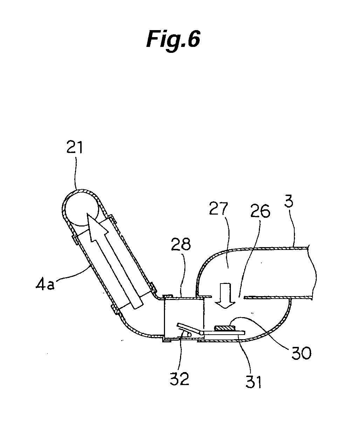

As shown in Fig. 6 which is a cross-sectional detail view of the lower side of the return duct (4a) in the coupling duct (4), a curved surface is formed at a lower end of the return duct (4a), and is coupled with the return port (26) of the bedding body (3) in a horizontal state via a rigid cylindrical body (28) provided so as not to be crushed even if stepped on, and the temperature sensor (30) is disposed on the bottom face of the return port (26) which is a flexible structure.

The temperature sensor (30) is disposed in a horizontal state, and is adapted such that the return air from above the return port (26) is vertically blown against the temperature sensor (30), more exact temperature detection is allowed, and breakage is prevented by a flexible structure even if stepped on, by increasing the area of contact of the return air with a sensor part.

The temperature sensor (30) detects the air temperature which has flowed through the air flow passage (27) of the bedding body (3) and has reached the return port (26), and controls the operation of the compressor (9) in the temperature control unit (2) so as to supply the air cooled or warmed to a user's desired temperature into the air flow passage (27), and exactly detects the air temperature which flows through the air flow passage (27), thereby controlling the inside of a sleeping space in an always comfortable temperature state, even if there are various load fluctuations, such as heat leakage from the outer surface of the bedding body (3) caused by the ambient air and the heat generation load caused by the user's body temperature.

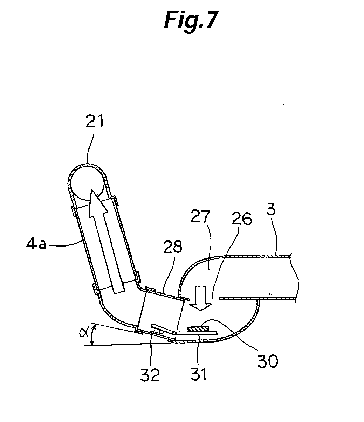

The temperature sensor (30) is locked to a plate-like sensor fixture (31). The sensor fixture (31) has one end rotatably supported at the end of the cylindrical body (28), and is adapted to hold its horizontal state by always pushing a locking portion of the temperature sensor (31) against the bottom face of the return port (26) by a spring (32). As the position or spacing from the temperature control unit (2) are changed depending on the installation condition of the bedding body (3) that is a flexible member, for example, as shown in Fig. 7, the inclination angle (α) of the return duct (4a) changes. Even when the spacing between the temperature control unit (2) and the bedding body (3) becomes narrow, and the horizontal state of the cylindrical body (28) cannot be maintained, the sensor fixture (31) is pushed against the bedding body (3) by a pivot portion and spring action thereof so that the horizontal state of the temperature sensor (30) positioned in the return port (26) can be maintained, and the temperature detection of the return air can be kept from varying and becoming unstable.

In addition, the position where the temperature sensor (30) is installed is not limited to the above place. For example, as shown in Fig. 2, the temperature sensor may be provided near the return air trunk (21) of the temperature control chamber (17) in the temperature control unit (2). In this case, the wiring configuration or work of the temperature sensor (30) can be more simply performed.

An upper portion of the heat-insulating wall (6) of the top face of the temperature control chamber (17) is used as a housing portion (33) for electrical components, such as a board, and a control panel (34) is arranged at the front face of the temperature control chamber so as to control the operation of the temperature control unit (2).

For example, when a cooling operation is performed, the temperature control can be made by a high-capability and high-efficiency cooling operation by performing such circulation that temperature-controlled cool air is generated by controlling flow passages by the four-way valve (14) of the refrigeration cycle (8) in the temperature control unit (2) so that the refrigerant from the compressor (9) is discharged to the condenser (10) and is evaporated in the evaporator (13), and this cool air is sent to the air flow passage (27) via the blowoff duct (4b) and the intake port (25) of the bedding body (3) from the blowoff air trunk (22) by the blower fan (18), cools the inside of the bedding body (3), and returns to the evaporator (13) in the temperature control chamber (17) via the return duct (4a) from the return port (26). Further, in case of a warming operation, the temperature of air is made high by switching the evaporator (13) to the condensation side by the four-way valve (14), and circulation air is warmed by heat exchange so as to be blown off toward the bedding body (3).

The bedding body (3) can obtain the temperature control effects for both cooling and warming by the employment of the four-way valve (14). However, when only the cooling action is used, a refrigeration cycle from which the four-way valve (14) is removed may be employed, and a stirring refrigerator may be used as a refrigerating machine.

By the above configuration, temperature controlled cool air or warm air is supplied to the bedding body (3) on the basis of the user's setting by the control panel (34) of the temperature control unit (2). Thus, an always comfortable sleeping space can be provided even under a variety of used environment conditions. If the installation space of the bedding body (3) is in an automobile, the cooling or warming capability of the whole cabin is not required, but it is sufficient if only the bedding body (3) is cooled or warmed. Therefore, the power consumption becomes minimum and economical, and the effect that the amount of emission of carbon dioxide gas can also be greatly suppressed by stopping idling is exhibited.

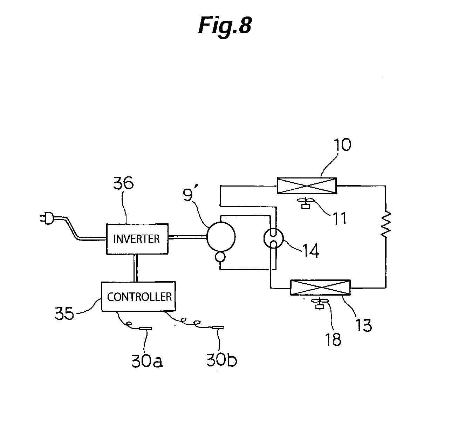

In addition, as shown in Fig. 8, the capability of the compressor (9') may be made variable, the temperature of respective places may be detected using as the two temperature sensors including a temperature sensor (30a) which detects the temperature of the return port (26) of the bedding body (3) and an ambient air temperature sensor (30a), the suitable operation frequency of a compressor (9') may be calculated by a controller (35) from the difference between the detection temperatures and the setting temperature of the bedding body (3), and the compressor (9') may be run by an inverter (36) on the basis of the command of the frequency.

Through such control, the operation of the compressor (9') according to a temperature load is allowed. Further, by almost eliminating temperature fluctuations within the bedding body (3), a more comfortable sleeping environment can be obtained, and cooling or warming can be rapidly performed by a high-capability operation at the time of initial operation. Additionally, the intermittent operation of the compressor (9') can be reduced, harsh noises during sleep can be suppressed, and consumed electric power can be reduced as operational loss decreases.

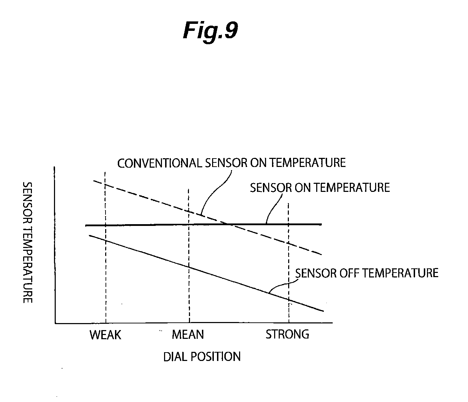

Additionally, as can be understood from Fig. 9 in which sensor temperature is shown on the vertical axis, and temperature control dial positions are shown on the horizontal axis, the operating characteristics of the temperature sensor (30) which detects the temperature in the air flow passage (27) of the bedding body (3) are as follows, for example. That is, in case of a cooling operation, the ON temperature of the temperature sensor (30) may be made constant; when the temperature of the air flow passage (27) has risen to a predetermined temperature, the compressor (9) may be driven irrespective of a dial position so as to make the temperature control unit (2) perform a cooling operation; and when the dial position is directed to "strong," the OFF temperature may be lowered accordingly so that the cooling degree is strengthened.

In case of a warming operation, although not particularly shown, contrary to the above, the OFF temperature of the temperature sensor (30) may be made constant, and the ON temperature may be made high along a dial plate position. If the control is made as described above, the operation of the bedding body (3) with low temperature fluctuation can be performed by the temperature control unit (2).

If the controller of the temperature control unit (2) adopts a remote control method by radio using an infrared ray, and is set in a suitable place around the bedding body (3), the user can control operation easily in a state where the user enters the bedding body (3) and lies down, and the operation may be controlled by a voice using a microphone.

Moreover, if sleep detecting sensors through the measurement of brain waves, and pulse waves or cardiac beats which have waveforms according to pressure changes in the blood extruded from the heart, and electrocardiographic measurement are used, and the control is performed such that a little strong cooling is made at the onset of sleeping and the temperature is rather raised after the onset of sleep, while the sleeping state of a person in the bedding is detected, a finer temperature atmosphere can be formed, and not only comfortable sleep but rising time can be controlled.

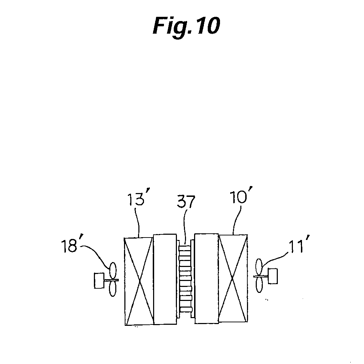

The following configuration may be adopted instead of the refrigeration cycle of the temperature control unit (2) in the above embodiment. That is, as shown in Fig. 10, a thermo-module (37) including a Peltier device that is a thermoelectric transducer is used, a heat exchanger (13') for cooling and a blower fan (18') are arranged on one side of the thermo-module, a heat exchanger (10') for heat radiation and a radiation fan (11') are arranged on the other side of the thermo-module, and a direct current voltage is applied to a thermo-module (37), to drive the respective fans (18') (11'), whereby the heat of the heat exchanger (13') for cooling cooled by an electronic cooling action is supplied to the bedding body (3) via the coupling duct (4) in the above embodiment so as to cool the bedding body to a predetermined temperature. When the bedding body (3) is warmed, switching can be made by reversing the plus and minus of an applied voltage to the thermo-module (37).

In this configuration, the temperature control unit (2) is simpler, there is no driving part in which the compressor is not used, silencing can be achieved, weight saving can be achieved, and a unit which is strong against vibration can be obtained.

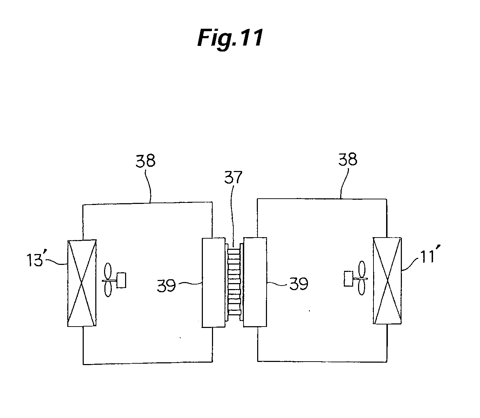

Additionally, as shown in Fig. 11, in addition to the thermo-module (37), if a thermo-siphon (38) which encloses a working fluid inside tubes and performs heat transfer by a change of phase is used so that the heat of the thermo-module (37) is transferred to the respective heat exchangers (11') (13') via a heat transfer block (39) (39) by the thermo-siphon (38), in addition to the effects of the above embodiment, the arrangement layout of components in the temperature control unit (2) and the length of the coupling duct (4) can be shortened or omitted, and cost reduction can be achieved.

In addition, although the temperature-controlled air circulation type bedding (1) including the temperature control unit (2) and bedding body (3) to be used in vehicles, such as a truck, has been described in the above respective embodiments, the invention is not limited to the vehicle-mounted bedding, and can also be utilized not only for individuals at home but for hospitals, and can also be widely spread and used for outdoors. As long as a case where these objects are used in the open air is taken into consideration, not only an alternator but a battery may be used as a power source.

### Brief Description of the Drawings

Fig. 1 is a schematic perspective view of temperature-controlled air circulation type bedding showing one embodiment of the invention.
Fig. 2 is a sectional view from the front of a temperature control unit of Fig. 1.
Fig. 3 is a side sectional view of Fig. 2.
Fig. 4 is a schematic configuration view of a refrigeration cycle disposed in Fig. 2.
Fig. 5 is a perspective view of the chief parts of Fig. 1.
Fig. 6 is an enlarged sectional view of the lower side of a return duct in a coupling duct of Fig. 5.
Fig. 7 is a sectional view showing a state where the inclination angle of the return duct in Fig. 6 has been changed.
Fig. 8 is a cycle configuration view using a capability variable compressor corresponding to Fig. 4.
Fig. 9 is a characteristic graph of a temperature sensor showing another embodiment of the invention.
Fig. 10 shows an embodiment according to a thermo-module of the temperature control unit of the invention.
Fig. 11 is an embodiment according to a thermo-siphon of the temperature control unit of the invention.
Fig. 12 is a sectional view showing a sleeping bag of a conventional example of the invention.
Fig. 13 is a sectional view of an air circulation type mat showing another conventional example of the invention.

### Description of Reference Numerals and Signs

- 1:: TEMPERATURE-CONTROLLED AIR CIRCULATION TYPE BEDDING
- 2:: TEMPERATURE CONTROL UNIT
- 3:: BEDDING BODY
- 4:: COUPLING DUCT
- 4a:: RETURN DUCT
- 4b:: BLOWOFF DUCT
- 5:: OUTER PLATE
- 5a:: BOTTOM PLATE
- 5b:: SUCTION OPENING
- 6:: HEAT-INSULATING WALL
- 6a:: HEAT-INSULATING PARTITION WALL
- 6b:: HEAT-INSULATING SIDE WALL
- 7:: MACHINE CHAMBER
- 8:: REFRIGERATION CYCLE
- 9, 9':: COMPRESSOR
- 10, 10':: CONDENSER
- 11, 11':: RADIATION FAN
- 13, 13':: EVAPORATOR
- 14:: FOUR-WAY VALVE
- 16:: FILTER
- 17:: TEMPERATURE-CONTROLLED CHAMBER
- 18:: BLOWER FAN
- 19:: CASING
- 20:: GUTTER PORTION
- 21:: RETURN AIR TRUNK
- 22:: BLOWOFF AIR TRUNK
- 25:: INTAKE PORT
- 26:: RETURN PORT
- 27:: AIR FLOW PASSAGE
- 27a:: BOTTOM FACE FLOW PASSAGE
- 27b:: TOP FACE FLOW PASSAGE
- 27c:: LOWER FLOW PASSAGE OF SIDE WALL
- 27d:: UPPER FLOW PASSAGE OF SIDE WALL
- 28:: SPACE
- 30, 30a, 30b:: TEMPERATURE SENSOR
- 31:: SENSOR FIXTURE
- 32:: SPRING
- 33:: ELECTRICAL COMPONENT HOUSING PORTION
- 34:: CONTROL PANEL
- 35:: CONTROLLER
- 36:: INVERTER
- 37:: THERMO-MODULE
- 38:: THERMO-SIPHON
- 39:: HEAT TRANSFER BLOCK

## Claims

1. Temperature-controlled air circulation type bedding comprising a temperature control unit which controls the blowoff air temperature by a cooling or heating action, and a bedding body which provides an air flow passage which allows the blowoff air from the temperature control unit to be introduced and circulated therethrough, and cools or warms the inside thereof,
wherein the temperature of air which circulates through the bedding is detected so that the temperature of the blowoff air is controlled by the temperature control unit.

2. The temperature-controlled air circulation type bedding according to Claim 1,
wherein a sensor which detects air temperature is attached between an outlet of the air flow passage and the temperature control unit in the bedding body, and the operation control of the temperature control unit is performed by the detection temperature by the sensor.

3. The temperature-controlled air circulation type bedding according to Claim 2,
wherein a sensor which detects air temperature is installed in an air trunk for return air of the temperature control unit.

4. The temperature-controlled air circulation type bedding according to Claim 2,
wherein the bedding body and the temperature control unit are coupled together by a coupling duct, the coupling duct is formed by a return duct to the temperature control unit from the bedding body and by a blowoff duct to the bedding body from the temperature control unit, and the sensor which detects air temperature is installed in the return duct.

5. The temperature-controlled air circulation type bedding according to Claim 4,
wherein the coupling duct is formed from a cylindrical rigid body, a connecting portion of the bedding body to the coupling duct is formed from an elastic material which has flexibility, a sensor is horizontally installed so as to become vertical to the flow of air to a discharge side in the connecting portion, and the sensor is attached to a sensor fixture which holds a holding portion rotatably at an end of the coupling duct.

6. The temperature-controlled air circulation type bedding according to Claim 2,
wherein the ON temperature of the sensor which detects the outlet temperature of the air flow passage is held at a constant value, and the OFF temperature of the sensor is changed by a temperature control dial.

7. The temperature-controlled air circulation type bedding according to Claim 1,
wherein a refrigeration cycle which connects a compressor, a condenser, and an evaporator annularly constitutes a temperature control unit, the evaporator is formed from a heat-insulating wall body and is housed in a temperature control chamber which has a blowoff air trunk and a return air trunk, and the compressor and the condenser are disposed in a machine chamber, and the evaporator is used as a heat exchanger capable of switching cooling and warming by switching refrigerant flow passages by a four-way valve provided so as to connect the upstream and downstream of the compressor.

8. The temperature-controlled air circulation type bedding according to Claim 7,
wherein a capability variable type by an inverter is used as the compressor, and the temperature of the vaporizer is controlled by detecting the outlet temperature of the air flow passage in the bedding body and ambient temperature, thereby controlling the number of revolutions of the compressor.
